# EUROPEAN PATENT APPLICATION

(11) **EP 2 177 207 A2**
(43) Date of publication of application: **21.04.2010**
(21) Application number: 09013001.4
(22) Date of filing: 14.10.2009
(51) Int. Cl.: A61K 8/46, A61K 8/87, A61Q 5/02, A61Q 19/10

(54) **Fatty acid-free, foaming cosmetic composition and method of use**

(30) Priority: 15.10.2008 US 196293 P
(71) Applicant: L'Oréal, 75008 Paris (FR)
(72) Inventor: Fares, Hani, Somerset, New Jersey 08873 (US); Russell, Michael, Chatham, New Jersey 07928 (US); Cornell, Marc, Jackson, New Jersey 08527 (US)
(74) Representative: Blodig, Wolfgang

(57) **Abstract**

Disclosed are cosmetic compositions containing a disodium lauryl sulfosuccinate and a hydrophilically modified urethane. Also disclosed are methods for treating a keratinous substrate, such as skin or hair, by applying the cosmetic compositions to the keratinous substrate to, e.g., cleanse the keratinous substrate or deposit a cosmetically acceptable ingredient thereupon.

## Description

### BACKGROUND OF THE INVENTION

Cosmetic products, and particularly cosmetic products such as cleansers, have been developed with the aim to provide a foaming action upon application to skin or hair. Some of these products require large amounts of water to produce a foam or have a pH much higher than that of skin or hair. In addition, currently available products which require less water ("low-water products") are typically unstable, undergoing undesirable phase separation during storage, and thus require mixing before application.

### SUMMARY OF THE INVENTION

A first aspect of the present invention is directed to a fatty acid-free, foaming cosmetic composition comprising a disodium lauryl sulfosuccinate and a hydrophilically modified polyurethane. In various embodiments, the disodium lauryl sulfosuccinate may be present in an amount from about 20 wt% to about 40 wt% based on the total weight of the composition; the hydrophilically-modified urethane may be present in an amount from about 1 wt% to about 5 wt% based on the the total weight of the composition; the pH of the composition may be from about 5.2 to 5.8. In additional embodiments, the amount of water present in the compositions of the present invention may be less than about 20 wt% based on the total weight of the composition;

Another aspect of the present invention is directed to a method for treating a keratinous substrate comprising applying to the keratinous substrate a fatty acid-free, foaming cosmetic composition containing a disodium lauryl sulfosuccinate and a hydrophilically modified polyurethane as contemplated in the various embodiments described above.

Underlying the present invention is the discovery that the combination of a disodium lauryl sulfosuccinate and a hydrophilically modified urethane produce a cosmetic composition that exhibits a foaming action when applied to the skin or hair without the need for large amounts of water unlike other foaming compositions commonly used in the cosmetics industry.

Applicants have also discovered that the inventive compositions may be formulated at a pH much lower than other foaming compositions commonly used in the cosmetics industry. Because the inventive compositions have a pH close to or the same as that of skin or hair they tend to produce less damage to the skin or hair.

Applicants have further discovered that the inventive compositions are stable, i.e., do not split or separate into different phases, over a much longer period of time compared to other low-water, foaming compositions currently available in the cosmetics industry.

### DETAILED DESCRIPTION

As contemplated herein, the compositions of the present invention comprise succinates as an anionic detersive surfactant. Suitable surfactants include, but are not limited to, mono- and di-esters of sulfosuccinates, e.g., saturated and unsaturated C₁₂₋₁₈ monoester sulfosuccinates, such as the disodium lauryl sulfosuccinate commercially available as MACKANATE™ LO (McIntyre Group, Ltd., University Park, IL). MACKANATE™ LO as commercially available is a high solids surfactant containing water and about 40% solids in a combination of disodium lauryl sulfosuccinate, trisodium sulfosuccinate, lauryl alcohol, and sodium sulfate in equilibrium in paste form. As understood by one of skill in the art, however, other forms of succinates, including disodium lauryl sulfosuccinate, are available (e.g. powders) and may also be employed in the present invention where appropriate.

It is contemplated herein that the anionic detersive succinate, e.g., MACKANATE™ LO, may be present in the compositions of the present invention in an amount that generally ranges from about 10% to about 60%, and in some embodiments, from about 15% to about 45%, and yet other embodiments from about 20% to about 30%, wherein all percentages are based on the total weight of the cosmetic composition. In a particular embodiment, the amount of succinate surfactant is about 28.15% of the total weight of the composition.

Polyurethanes are polymers consisting of a chain of organic units joined by urethane links. Polyurethane polymers are formed by reacting a monomer containing at least two isocyanate functional groups with another monomer containing at least two alcohol groups (i.e., a diol or polyol) in the presence of a catalyst. Simple polyurethanes are insoluble in water. As used herein, the term "hydrophilically modified polyurethane" means a polyurethane in which the monomers used have been chosen or modified to make the polyurethane much more soluble in water.

For example, an alcohol monomer that has been ethoxylated may be used to produce a hydrophilically modified polyurethane. A suitable example of such compound includes compounds of the below formula (I):

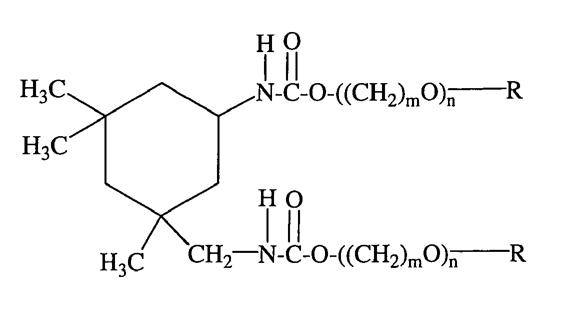

where n is a whole number between about 50 and about 150, preferably between about 70 and about 120, and most preferably between about 75 and 100, including all ranges and subranges therebetween; m is a whole number between 1 and 5, preferably 2 and/or 3 (ethoxylation and/or propoxylation), and most preferably 2 (ethoxylation); and R represents a C₁₂-C₂₄ alkyl or alkenyl fatty portion, preferably a C₁₄-C₂₂ fatty portion, and most preferably a a C₁₆-C₁₈ fatty portion. Preferred fatty alkoxylated dimeric compounds are compounds comprising between about 75 and about 100 ethoxylated units and a C₁₆-C₁₈ fatty portion. Particularly preferred examples of such compounds are compounds having 75 or 100 mole (or units) of ethoxylation marketed under the tradenames Dermothix 75 (INCI name Distearth-75 IPDI) and Dermothix 100 (INCI name Distearth-100 IPDI), respectively (Alzo International, Inc., Sayerville, NJ), which are polyurethanes based on an ethoxylated stearyl alcohol.

The hydrophilically modified urethane is present in an amount that generally ranges from about 0% to about 20%, and in some embodiments, from about 0.5% to about 10%, and yet other embodiments from about 1% to about 5%, wherein all percentages are based on the total weight of the cosmetic composition. In some other embodiments, the amount of hydrophilically modified polyurethane is about 2% of the total weight of the composition.

In an embodiment of the present invention, the cosmetic composition is a paste but gels, milks, creams, ointments, mousse forms, or lotions are also contemplated herein. In an embodiment of the present invention, the cosmetic compositions are white and/or pearly in appearance and have a thick, creamy, structured feel. In an embodiment of the present invention, the cosmetic composition may be in the form of a cleanser, e.g., a shampoo, face wash, body wash, night cream, or the like.

In an embodiment of the present invention, the cosmetic composition has a viscosity of from about 50,000 centipoises to about 250,000 centipoises, preferably about 150,000 to about 200,000 centipoises. (Brookfield Method ("BF") (Spindle T-D) 20°C.)

In an embodiment of the present invention, the cosmetic composition has a pH of from about 4.5 to about 6.5, preferably from about 5.2 to about 5.8, more preferably about 5.5.

The cosmetic compositions of the present invention may be applied to keratinous substrates (tissue or synthetic), particularly skin and hair. The compositions of the present invention may be applied by hand. Alternatively, or in conjunction therewith, they may be applied via an applicator such as a sponge, cotton, brush or a puff of a natural or synthetic material. In addition, the applicator may be attached to a container, the container serving as a reservoir for the cosmetic composition.

When applied to the keratinous substrate with lathering, *e.g.,* with a rubbing or massaging action, the cosmetic compositions of the present invention produce a foam. In an embodiment of the present invention, the foam produced by lathering the cosmetic composition has a texture similar to that of a shaving cream. In an embodiment of the present invention, the cosmetic composition, upon lathering, produces a foam having a height greater then or equal to about 300 millimeters, preferably greater than or equal to about 390 millimeters. Foam height is measured according to the Ross-Miles method (NF T 73-404/IS696).

In an embodiment of the present invention, the cosmetic composition, upon lathering, produces a foam having a foam scale score of from about 2.5 to about 4.5, preferably from about 3 to about 4. As used herein, foam scale is an observational determination of the firmness/structure of the foam on a scale of 1 to 5. A 1 on the foam scale is a foam that has little or no firmness/structure, *e.g.,* dish soap bubbles. A 5 on the foam scale is a highly structured, firm foam, *e.g.,* jelly-like.

As used herein, the term "low water" product in the context of a cosmetic composition of the present invention refers to a composition which does not require copious amounts of water to bring forth the desired foaming or lathering of the composition. Contemplated volumes of water for use with the compositions and methods of the present invention are less than typically necessary to achieve a desired amount of foaming or lathering with compositions which would not be considered a "low water" product. Suitable amounts of water for use with the present invention may be determined by one of skill in the art.

The cosmetic compositions may further comprise at least one additional cosmetically acceptable ingredient. Examples of such ingredients are familiar to one of skill in the art and include solvents, emulsifiers, surfactants, structuring agents such as waxes and polymers, hydrophobic (lipophilic) and hydrophilic thickeners or gelling agents, skin conditioning agents (humectants, exfoliants or emollients), fillers (*e.g.,* powders and mother-of-pearl), fibers, sunscreen agents (*e.g.,* octocrylene, octinoxate, avobenzone), preservatives *(e.g.,* sodium citrate, phenoxyethanol, parabens and mixtures thereof), chelators (such as EDTA and salts thereof, particularly sodium and potassium salts), neutralizing or pH-adjusting agents (*e.g.,* sodium hydroxide), cosmetically active agents and dermatological active agents such as, for example, additional skin care actives such as peptides (*e.g.,* pentapeptide derivatives such as palmitoyl pentapeptide-4 or MATRIXYL (Procter & Gamble) farnesol, bisabolol, phytantriol, vitamins and derivatives thereof such as ascorbic acid, vitamin A (*e.g.,* retinoid derivatives such as retinyl palmitate or retinyl propionate), vitamin E (*e.g.,* tocopherol acetate), vitamin B₃ (*e.g.,* niacinamide) and vitamin B₅ (*e.g.,* panthenol), anti-acne agents (resorcinol and salicylic acid), antioxidants (*e.g.,* phytosterols, BHT, tocopherol and lipoic acid), flavonoids (*e.g.,* isoflavones, phytoestrogens), colorants, aesthetic agents such as essential oils, fragrances, skin sensates, opacifiers, aromatic compounds (*e.g.,* clove oil, menthol, camphor, eucalyptus oil, and eugenol), foam enhancers, botanical extracts, and anti-inflammatory agents.

The compositions of the present invention may contain a solvent. The solvent may be polar or non-polar, volatile or non-volatile, or aqueous or non-aqueous in nature. Representative volatile solvents include non-polar volatile hydrocarbon-based oils (which as used herein, refers to oil containing only hydrogen and carbon atoms), silicone oils (optionally comprising alkyl or alkoxy groups that are pendant or at the end of a silicone chain), and fluoro oils. Suitable hydrocarbon-based oils include isoparaffins, i.e., branched alkanes containing 8-16 carbon atoms, such as isododecane (also known as 2,2,4,4,6-pentamethylheptane), and petroleum distillates. Suitable silicone oils may include linear or cyclic silicones containing from 2 to 7 silicon atoms, these silicones optionally comprising alkyl or alkoxy groups containing from 1 to 10 carbon atoms. Examples include octamethylcyclotetrasiloxane, decamethylcyclopentasiloxane, hexadecamethylcyclohexasiloxane, heptamethylhexyltrisiloxane and heptamethyloctyltrisiloxane, and mixtures thereof. Mixtures of these solvents may be used. Polar volatile solvents may also be used, examples of which include C₂ to C₅ alcohols, such as ethanol, ethyl 3-ethoxypropionate and isohexyl neopentanoate.

Exemplary non-polar non-volatile solvents include polyalphaolefins, which include ethylene derivatives oligomerized into even-numbered carbon polyalphaolefins *e.g.,* C₆-C₁₄ olefins such as polydecene and polymers of C₆, C₈, C₁₂ and C14 olefins. The polyolefins may have a molecular weight (MW) generally ranging from about 280 to about 11,500, and a viscosity (cps at about 20°C) generally ranging from about 7 to about 32,500. They may also be hydrogenated. In some embodiments, the non-volatile solvent includes PureSyn^{™}2 (MW about 283), 4 (MW about 432), 6 (MW about 570), 8 (MW about 611), 150 (MW about 3980) and 300 (MW about 4870) (INCI name: hydrogenated polydecene). The viscosity of these polymers is about 8, about 33, about 64, about 103, about 4179 and about 8400, respectively.) PureSyn^{™} 100 (MW about 2939, viscosity about 3900, INCI name: hydrogenated C6-14 olefin polymers) and PureSyn^{™} 1000 (MW about 11,500, viscosity about 32,400, INCI name: polydecene) may also be useful. The PureSyn^{™} products are available from Exxon Chemicals.

The compositions of the present invention may contain emulsifiers such as anionic, nonionic and cationic emulsifiers. See, *e.g.,* Encyclopedia of Chemical Technology, KIRK-OTHMER, volume 22, pp. 333-432, 3rd edition, 1979, Wiley, for the definition of the properties and (emulsifying) functions of the emulsifiers, in particular pp. 347-377 of this publication regarding anionic and nonionic emulsifiers. Examples of emulsifiers useful in the compositions of the invention include as nonionic emulsifiers, fatty acids, fatty alcohols, polyethoxylated fatty alcohols or polyglycerolated fatty alcohols, such as polyethoxylated stearyl alcohols or cetylstearyl alcohols, esters of fatty acid and sucrose, and glucose alkyl esters, in particular polyoxyethylenated C₁-C₆ alkyl glucose fatty esters, and as anionic emulsifiers, C₁₆-C₃₀ fatty acids neutralized by amines, ammonia or the alkali metal salts thereof. Examples of cationic emulsifiers include quaternary amines, amine oxides and amines, *e.g.,* alkyl amines, alkyl imidazolines, ethoxylated amines, quaternary compounds, and quaternized esters. Cationic emulsifiers may also provide a conditioning effect.

The compositions may contain additional surfactants such as, for example, a nonionic surfactant, an anionic surfactant, an amphoteric/zwitterionic surfactant, and a cationic surfactant. Examples of suitable nonionic surfactants include, but are not limited to, fatty acid esters and alkoxylated, particularly ethoxylated, fatty acid esters of polyhydric alcohols such as glycerols and sorbitol, for example, polyoxyethylene monolaurate, polyoxyethylene monooleate, polyoxyethylene monostearate, sorbitan monolaurate, sorbitan trioleate, generally with a degree of ethoxylation of from about 20 to about 85; mono- and dialkanolamides, such as the N-acyl derivatives of mono- and diethanol amines, and polyethoxylated monoalkanolamides; amine oxides, such as cocoamidopropyl dimethylamine oxides, coco bis-2-hydroxyethyl amine oxides and lauryl dimmethylamine oxide; ethoxylated alkanolamides; ethoxylated oils and fats such as ethoxylated lanolins; and ethoxylated alkylphenols, such as Nonoxynol.

Suitable anionic surfactants include, for example, the following: the alkali metal, ammonium, or amine salts of alkyl sulfates, alkyl ether sulfates, linear alpha-olefin sulfonates, dialkyl sulfosuccinates, alkylamidosulfosuccinates, and alkyl taurates each having from about C₁₂ to C₁₈ alkyl or alkenyl groups. Particular examples include the salts of lauryl sulfates and lauryl ether sulfates the latter having an average level of ethoxylation of 1-3.

Amphoteric/zwitterionic surfactants belong to the category of surface active chemicals that possess a positive and a negative charge in the same molecule and behave as a cation or an anion depending on the pH of the medium. In general, the positive charge is located on a nitrogen atom while the negative charge is carried by a carboxyl or sulfonate group.

Suitable amphoteric surfactants include, for example, lauryl betaine, lauroamphoglycinate, lauroamphopropylsulfonate, lauroamphopropionate, lauroamphocarboxyglycinate, lauryl sultane, myristamidopropyl betaine, myristyl betaine, myristoamphoglycinate, myristyl propionate, stearoamphoglycinate, stearoamphopropionate, stearoamphopropylsulfonate, stearyl betaine, cocamidoethyl betaine, cocamidopropyl betaine, cocamidopropyl hydroxysultane, cocamidopropyl dimethylamine propionate, cocoamphoglycinate, cocoamphocarboxypropionate, cocoamphocarboxyglycinate, coco-betaine, cocoamphopropionate, cocoamphopropylsulfonate.

Examples of amphoteric surfactants for use in this invention include: cocamidopropyl betaine, coco-betaine, stearyl betaine, cocoamphocarboxyglycinate, cocoamphodipropionate, and stearoamphoglycinate.

The compositions may contain structuring agents such as a wax, a polymer, a thickener, or a gelling agent. For the purposes of the present invention, the term "wax" means a lipophilic fatty compound that is solid at room temperature about (25ºC) and atmospheric pressure (760 mmHg, i.e., 105 Pa), which undergoes a reversible solid/liquid change of state and which has a melting point of greater than 30ºC and in some embodiments, greater than about 55ºC up to about 120ºC or even as high as about 200ºC. For the purposes of the invention, the waxes are those generally used in cosmetics and dermatology. A variety of waxes may be useful, including waxes of animal origin, waxes of plant origin, waxes of mineral origin and waxes of synthetic origin. Examples of waxes of animal origin include beeswaxes, lanolin waxes and Chinese insect waxes. Examples of waxes of plant origin include rice waxes, carnauba wax, candelilla wax, ouricurry wax, cork fibre waxes, sugar cane waxes, Japan waxes, sumach wax and cotton wax. Examples of waxes of mineral origin include paraffins, microcrystalline waxes, montan waxes and ozokerites. Examples of waxes of synthetic origin include polyolefin waxes, *e.g.,* polyethylene waxes, waxes obtained by Fischer-Tropsch synthesis, waxy copolymers and their esters, and silicone and fluoro waxes. Alternatively, hydrogenated oils of animal or plant origin may be used. Examples include hydrogenated jojoba waxes and hydrogenated oils which are obtained by catalytic hydrogenation of fats composed of a C₈-C₃₂ linear or nonlinear fatty chain, hydrogenated sunflower oil, hydrogenated castor oil, hydrogenated copra oil, hydrogenated lanolin and hydrogenated palm oils. In some embodiments, the compositions contain at least two or at least three waxes.

Examples of suitable polymers include polyvinylpyrrolidones (PVP) and vinyl copolymers, *e.g.,* vinyl pyrrolidone (VP)/hexadecane copolymer, PVP/hexadecene copolymer and VP/eicosene copolymer (*e.g.,* Ganex V220, which is a tradename of ISP Inc. of Wayne, NJ), trimethylsiloxysilicate and acrylates copolymer.

Examples of suitable lipophilic thickeners or gelling agents include modified clays such as hectorites modified with an ammonium chloride of a C₁₀ to C₂₂ fatty acid, such as hectorite modified with distearyldimethylammonium chloride, also known as quaternium-18 bentonite, such as the products sold or made under the names Bentone 34 by the company Rheox, Claytone XL, Claytone 34 and Claytone 40 sold or made by the company Southern Clay, the modified clays known under the name quaternium-18 benzalkonium bentonites and sold or made under the names Claytone HT, Claytone GR and Claytone PS by the company Southern Clay, the clays modified with stearyldimethylbenzoylammonium chloride, known as stearalkonium bentonites, such as the products sold or made under the names Claytone APA and Claytone AF by the company Southern Clay, and Baragel 24 sold or made by the company Rheox, hydrophobic silica, such as fumed silica, metal salts of fatty acids and polyethylene.

Water-soluble or hydrophilic thickeners or gelling agents that may be used include carboxyvinyl polymers, such as polyvinylpyrrolidone (PVP) and polyvinyl alcohol, crosslinked acrylates (*e.g.* Carbopol 982), hydrophobically-modified acrylates (*e.g*. Carbopol 1382); polyacrylamides such as the crosslinked copolymers sold under the names Sepigel 305 (CTFA name: polyacrylamide/C₁₃-C₁₄ isoparaffin/Laureth 7) or Simulgel 600 (CTFA name: acrylamide/sodium acryloyldimethyltaurate copolymer/isohexadecane/polysorbate 80) by SEPPIC; 2-acrylamido-2-methylpropanesulphonic acid polymers and copolymers, that are optionally crosslinked and/or neutralized; cellulose derivatives such as hydroxyethylcellulose, sodium carboxymethylcellulose, hydroxypropyl methylcellulose, hydroxypropyl cellulose, ethyl cellulose and hydroxymethyl cellulose, polysaccharides and natural gums such as xanthan gum, sclerotium, carrageenan and pectin, polysaccharide resins such as starch and its derivatives, and hyaluronic acid and its salts.

The compositions of the present invention may also contain skin conditioning agents (such as a humectants, exfoliant or emollients). Examples of suitable skin conditioning agents include sodium lactate, mannitol, amino acids, hyaluronic acid, lanolin, urea, petroleum jelly and mixtures thereof. Other examples include polyols such as glycerin, diglycerin, triglycerin, polyglycerin, polyethylene glycol and sorbitol. The C₂-C₈ glycols disclosed herein are also useful in this respect.

Fillers, powders, and mothers-of-pearl may also be present, typically to modify the texture of the composition and the matteness/gloss effect. Fillers should be understood to mean lamellar or non-lamellar, inorganic or synthetic, colorless or white particles. Mothers-of-pearl should be understood to mean iridescent particles produced especially by certain mollusks in their shell or else synthesized. Representative examples of these ingredients include mica, silica, kaolin, iron oxides, titanium dioxide, polyamide powders, polyamide powders, for instance Nylon® (Orgasol from Atochem), poly-alanine powders, polyethylene powders, tetrafluoroethylene polymer powders, for instance Teflon®, starch, boron nitride, hollow polymer microspheres such as those of polyvinylidene chloride/acrylonitrile, for instance Expancel® (Nobel Industrie), acrylic powders such as Polytrap® (Dow Corning), polymethyl methacrylates particles and silicone resin microbeads (for example Tospearls® from Toshiba), magnesium hydrocarbonate, hydroxyapatite, hollow silica microspheres (Silica Beads® from Maprecos), and glass and ceramic microcapsules.

The compositions of the present invention may also contain fibers, which may be chosen from natural and synthetic fibers. Natural fibers include, but are not limited to, cotton, silk, wool, and other keratin fibers. Synthetic fibers include, but are not limited to, polyester, rayon, nylon and other polyamide fibers.

Colorants may be chosen from the lipophilic dyes, hydrophilic dyes, traditional pigments, and nacres usually used in cosmetic or dermatological compositions, and mixtures thereof. The coloring agent may have any shape, such as, for example, spheroidal, oval, platelet, irregular, and mixtures thereof. Pigments may optionally be surface-treated *e.g.,* with silicones (*e.g.,* inorganic pigments may be coated with simethicone), perfluorinated compounds, lecithin, and amino acids.

The liposoluble dyes include, for example, Sudan Red, D&C Red 17, D&C Green 6, soybean oil, Sudan Brown, D&C Yellow 11, D&C Violet 2, D&C Orange 5, quinoline yellow and annatto. The water-soluble dyes are, for example, beetroot juice or methylene blue.

The pigments may be chosen from white pigments, colored pigments, inorganic pigments, organic pigments, coated pigments, uncoated pigments, pigments having a micron size and pigments not having a micron size. Among the inorganic pigments that may be mentioned are titanium dioxide, optionally surface-treated, zirconium oxide, zinc oxide, cerium oxide, chromium oxide, manganese violet, ultramarine blue, chromium hydrate, and ferric blue. Among the organic pigments which may be mentioned are carbon black, pigments of D&C type, lakes based on cochineal carmine, lakes based on barium, lakes based on strontium, lakes based on calcium, and lakes based on aluminum.

The nacreous pigments may, for example, be chosen from white nacreous pigments such as mica coated with titanium and mica coated with bismuth oxychloride, colored nacreous pigments such as titanium mica with iron oxides, titanium mica with, for example, ferric blue and/or chromium oxide, titanium mica with an organic pigment of the type mentioned above, as well as nacreous pigments based on bismuth oxychloride, interferential pigments, and goniochromatic pigments.

The compositions of the present invention may also include botanical extracts. Examples of botanical extracts include PURISOFT™ (peptide from Moringa seeds (Laboratoires Séroboilogiques, Pulnoy, France), lichoderm, EXFOLACTIVE EL™ (Hydrolyzed *Opuntia ficus indica* Flower extract, SILAB, Brive, France), MOIST 24™ (Imperata Cylindrica (root extract) formula, Sederma, Le-Perray-en-Yvelines, France), GLYCOLFILM™ (biosaccharide gum-4, Solabia, Pantin, France), and lemon peel extract.

It is contemplated herein that the present invention includes compositions comprising any and all possible combinations of ingredients described herein and methods of treating with said compositions.

The following examples are intended to further illustrate the present invention. They are not intended to limit the invention in any way. Where trade names for various ingredients are given, the percentage indicated reflects the weight used of the ingredient as it comes from the manufacturer and not just the active component in that ingredient.

EXAMPLES

Example 1

A cosmetic composition of the present invention, specifically a non-soap cleanser, is described below.

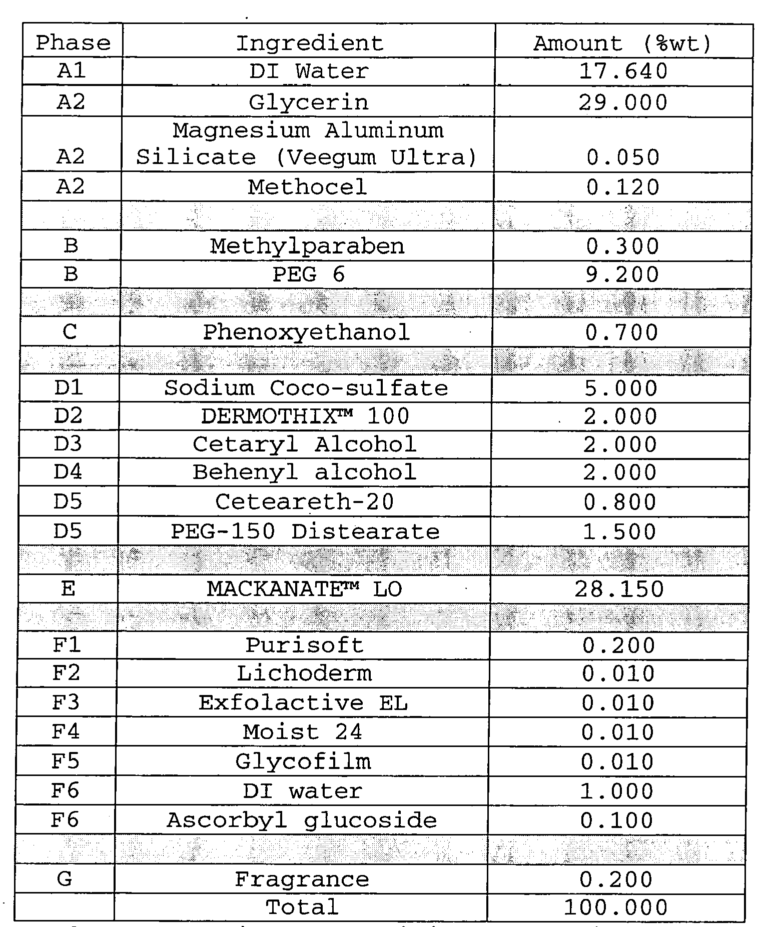

The cosmetic composition described above was prepared as follows. The Phase A1 ingredient (water) was added to a main kettle, followed by initiating of heating at about 75-80°C with moderate homogenization. Premixed Phase A2 ingredients were added to the main kettle with homogenization until uniform. While maintaining the batch temperature at about 75-80°C, premixed Phase B ingredients were added to the main kettle and mixed until uniform. The Phase C ingredient was added to the main kettle and mixed for 5 minutes until uniform, while again maintaining the batch temperature at about 75-80°C. Phase D ingredients were added one at a time with mixing until uniform with low to moderate homogenization and sweep mixing before addition of the next Phase D ingredient. The batch was then cooled to about 55-60ºC. The Phase E ingredient was added and mixed with homogenization and sweep mixing until uniform. The batch was then cooled to about 45ºC. The Phase F and G ingredients were then added with low homogenization and sweep mixing until uniform. Once the batch was uniform the mixing was switched to only low sweep mixing and a vacuum of 7-8 psi was applied to the batch. Low sweep mixing of the batch was continued under the vacuum until the batch cooled to room temperature.

The resulting cosmetic composition was a thick, white, smooth paste having a pH of 5.216 and a viscosity of 184,000 cps (BF (T-D) 20ºC). This paste upon lathering (*e.g.,* agitation, rubbing, massaging) produced a foam with a height of 390 millimeters and a 4 on the foam scale.

EXAMPLE 2

A cosmetic composition of the present invention, specifically a non-soap cleanser, is described below.

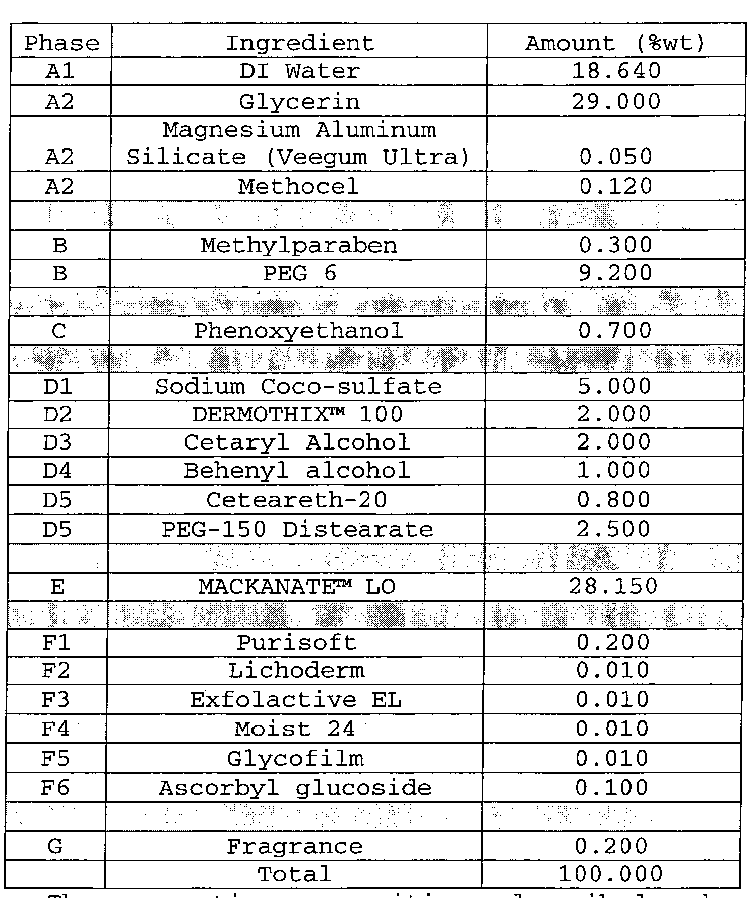

The cosmetic composition described above was prepared as follows. The Phase A2 ingredients were added to a main kettle and dispersed for 5 minutes and heating to 70-75ºC was begun. The Phase A1 ingredient (water) was added to the main kettle. While continuing to heat the batch to about 70-75°C, the Phase B and C ingredients were added to the main kettle. The temperature was stabilized at about 70-75ºC and the content of the main kettle mixed until homogenous (about 5 minutes). Phase D2-D5 ingredients were added to the main kettle and mixed for 10 minutes or until all waxes and powders are melted. Throughout this addition and mixing, the temperature was maintained at about 70-75°C. The Phase D1 ingredient was then added to the main kettle and dispersed for 10 minutes while maintaining the temperature at about 70-75°C. Without regulating temperature, the Phase E ingredient was added and mixed for 10 minutes. The mixture cooled to about 65ºC. While allowing the mixture to cool to about 25-30ºC, the Phase F and G ingredients were added. The mixture was then mixed for 2 minutes until smooth. The mixture was cooled to about 25-30ºC.

The resulting cosmetic composition was a thick, white, smooth paste having a pH of 5.216 and a viscosity of 170,000 cps (BF (T-D) 20ºC). This paste upon lathering (*e.g.,* agitation, rubbing, massaging) produced a foam with a height of 390 millimeters and a 4 on the foam scale.

Example 3 - Comparative Compositions

Compositions for comparison to the inventive compositions were prepared as follows. The cosmetic composition of Example 2 was modified by replacing the MACKANATE® LO with another surfactant. This substitution was carried out at a 1:1 ratio based on the amount of solids in the surfactant. The characteristics of these comparative compositions are shown below:

| **Surfactant** | **Charge** | **Phase separation** | **pH** | **viscosity (cps)** | **Appearance/Feel** | **Foam Scale** | **FoamHeight (mm)** |
|---|---|---|---|---|---|---|---|
| Disodium Ricinoleamido Mea-Sulfosuccinate | ANIONIC | Split | 6.294 | 25,000 (BF (T-C)) | Yellow, grainy. | 1 | 280 |
| Cocamidopropyl Betaine | AMPHOTERIC | Split | 7.559 | 59,000 (BF (T-C)) | Runny/viscous but stringy, waxy creamy. | 2 | 205 |
| Disodium Lauramido PEG-5 Sulfosuccinate | ANIONIC | Split | 5.992 | Low | Runny. | | 330 |
| Stearamine Oxide | CATIONIC | | 10.45 | Low | Spongy Texture. | 1.5 | 170 |
| Disodium Lauryl Sulfosuccinate (and) Sodium Cocoyl Isethionate (and) Sodium Laureth Sulfate | ANIONIC | | 9.706 | | Viscous paste-like matrix. | | 260 |
| Disodium Lauryl Sulfosuccinate | ANIONIC | | 6.99 | | White, creamy, smooth. | 2.5 | 315 |
| Sodium Laureth Sulfate | ANIONIC | Split | 8.746 | Low | Foamed but not creamy. | 3 | 400 |
| Disodium Lauryl Sulfosuccinate (and) Sodium Cocoyl Isethionate (and) Sodium Laureth Sulfate | ANIONIC | | 4.9 (pH adjusted with citric acid) | | | 1 | 315 |
| Disodium Cocoamphodiacetate | ANIONIC | Split | 8.7 | Low | | | |
| Lauramine Oxide | CATIONIC | | 6.2 | | Creamy, white, smooth until citric acid was added to adjust pH, then became clumpy, cottage cheese like. | 2 | 180 |
| Cocamidopropyl Betaine | AMPHOTERIC | Split | 7.13 | | | | |
| Setacin F | ANIONIC | Split | 4.961 | Low | Was rough to rub/almost scrub-like particles. | | |

As seen above, many of these comparative formulations have an unsatisfactory appearance or feel. Many also have a low foam height or low foam scale number, meaning that the foam produced on lathering is either runny or soupy. Finally, many of these comparative compositions undergo a split in the phases over a short storage time.

Example 4 - Comparative Composition

Another composition for comparison to the inventive compositions was prepared. This comparative composition was prepared by omitting the DERMOTHIX™ 100 from the cosmetic composition of Example 2. The comparative composition was much less structured than the pearly, very structured paste of Example 2 and had a viscosity of about one-third of that of the cosmetic composition of Example 2. The comparative composition was runny and had a much more liquid like texture.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

The content of US provisional patent application No. 61/196,293, filed on October 15, 2008, the priority of which is claimed by the present patent application, is herein incorporated by reference including description and all claims in its entirety.

## Claims

1. A fatty acid-free, foaming cosmetic composition comprising a disodium lauryl sulfosuccinate and a hydrophilically modified polyurethane.

2. The fatty acid-free, foaming cosmetic composition of claim 1, wherein said hydrophilically-modified polyurethane comprises DERMOTHIX™ 100.

3. The fatty acid-free, foaming cosmetic composition of claim 1, wherein said disodium lauryl sulfosuccinate comprises MACKANATE™ LO.

4. The fatty acid-free, foaming cosmetic composition of claim 1, wherein said disodium lauryl sulfosuccinate is present in an amount of about 20 wt% to about 40 wt%, based on the total weight of the composition.

5. The fatty acid-free, foaming cosmetic composition of claim 1, wherein said hydrophilically-modified polyurethane is present in an amount of about 1 wt% to about 5 wt%, based on the total weight of the composition.

6. The fatty acid-free, foaming cosmetic composition of claim 1, wherein the pH of the composition is from about 5.2 to about 5.8.

7. The fatty acid-free, foaming cosmetic composition of claim 1, wherein the amount of water present in the composition is less than about 20% based on the total weight of the composition.

8. The fatty acid-free, foaming cosmetic composition of claim 1, wherein the composition is a paste.

9. The fatty acid-free, foaming cosmetic composition of claim 1, wherein the composition, upon lathering, produces a foam with a foam height greater than about 350 millimeters.

10. The fatty acid-free, foaming cosmetic composition of claim 1, wherein the composition, upon lathering, produces a foam with a foam scale score greater then or equal to about 3.5.

11. The fatty acid-free, foaming cosmetic composition of claim 1, wherein the composition has a viscosity between about 50,000 and about 250,000 cps using the Brookfield Method with a T-D spindle at 20°C.

12. A fatty acid-free, foaming cosmetic composition comprising about 28.15 wt% MACKANATE™ LO and about 2 wt% DERMOTHIX™ 100, based on the total weight of the composition.

13. The fatty acid-free, foaming cosmetic composition of claim 1, further comprising at least one other cosmetically acceptable ingredient selected from the group consisting of solvents, emulsifiers, surfactants, structuring agents, thickeners or gelling agents, skin conditioning agents, fillers, fibers, sunscreen agents, preservatives, chelators, antioxidants, neutralizing or pH-adjusting agents, cosmetically active agents and dermatological active agents, flavonoids, colorants, aesthetic agents, foam enhancers, botanical extracts, anti-inflammatory agents, and mixtures thereof.

14. A method for treating a keratinous substrate such as skin or hair, comprising applying to the keratinous substrate a fatty acid-free, foaming cosmetic composition comprising a disodium lauryl sulfosuccinate surfactant and a hydrophilically modified polyurethane, optionally further comprising lathering the cosmetic composition.

15. The method of claim 14, wherein said method of treating comprises cleansing the keratinous substrate, or said method of treating comprises depositing a cosmetically acceptable ingredient on the surface of the keratinous substrate.
